(19) **Europäisches Patentamt / European Patent Office / Office européen des brevets**

(11) **EP 1 289 540 B1**

(12) # EUROPEAN PATENT SPECIFICATION

(45) Date of publication and mention
of the grant of the patent:
**07.03.2012 Bulletin 2012/10**

(21) Application number: **01946052.6**

(22) Date of filing: **01.06.2001**

(51) Int Cl.:
*A61K 38/18* (2006.01)      *A61P 25/00* (2006.01)
*A61K 38/18* (2006.01)      *A61K 31/00* (2006.01)

(86) International application number:
**PCT/US2001/017895**

(87) International publication number:
**WO 2001/091783 (06.12.2001 Gazette 2001/49)**

(54) **ONCOMODULIN FOR TREATING NEUROLOGICAL DISORDERS**

ONCOMODULIN ZUR BEHANDLUNG VON NEUROLOGISHEN ERKRANKUNGEN

ONCOMODULINE POUR LE TRAITEMENT DES TROUBLES NEUROLOGIQUES

(84) Designated Contracting States:
**AT BE CH CY DE DK ES FI FR GB GR IE IT LI LU MC NL PT SE TR**

(30) Priority: **01.06.2000 US 208778 P**

(43) Date of publication of application:
**12.03.2003 Bulletin 2003/11**

(73) Proprietor: **CHILDREN'S MEDICAL CENTER CORPORATION**
**Boston**
**Massachusetts 02115 (US)**

(72) Inventor: **BENOWITZ, Larry, I.**
**Newton, MA 02459 (US)**

(74) Representative: **Brown, David Leslie et al**
**Haseltine Lake LLP**
**Redcliff Quay**
**120 Redcliff Street**
**Bristol**
**BS1 6HU (GB)**

(56) References cited:
**WO-A-00/13705      WO-A-97/03188**
**WO-A-97/34618      FR-A- 2 720 069**

- **FLANDERS K C ET AL: "Transforming growth factor -betas in neurodegenerative disease." PROGRESS IN NEUROBIOLOGY, (1998 JAN) 54 (1) 71-85. REF: 101 , XP000857248**

- **DI IORIO, PATRIZIA ET AL: "Purine nucleosides protect injured neurons and stimulate neuronal regeneration by intracellular and membrane receptor-mediated mechanisms." DRUG DEVELOPMENT RESEARCH, (JAN FEB, 2001) VOL. 52, NO. 1-2, PP. 303-315. PRINT. , XP002196677**

- **POULSEN, K. T. ET AL: "TGF.beta.2 and TGF.beta.3 are potent survival factors for midbrain dopaminergic neurons" NEURON (1994), 13(5), 1245-52 , XP001073419**

- **KOEBERLE P D ET AL: "EFFECTS OF ANTI-INFLAMMATORY GENE TRANSFER ON THE SURVIVAL OF AXOTOMIZED RETINAL GANGLION CELLS IN VIVO: COMPARISON OF IL-10, IL-4 AND TGF-BETA", INVESTIGATIVE OPHTHALMOLOGY & VISUAL SCIENCE, ASSOCIATION FOR RESEARCH IN VISION AND OPHTHALMOLOGY, US, vol. 41, no. 4, 15 March 2000 (2000-03-15) , page S189, XP009031641, ISSN: 0146-0404**

- **FLANDERS K C ET AL: "TRANSFORMING GROWTH FACTOR-BETAS IN NEURODEGENERATIVE DISEASE", PROGRESS IN NEUROBIOLOGY, PERGAMON PRESS, GB, vol. 54, no. 1, 1 January 1998 (1998-01-01), pages 71-85, XP000857248, ISSN: 0301-0082, DOI: DOI: 10.1016/S0301-0082(97)00066-X**

Remarks:
The file contains technical information submitted after the application was filed and not included in this specification

**Description**

*Related Applications*

This application claims priority to U.S. Provisional Application No. 60/208,778, filed on June 1, 2000.

*Background of the Invention*

Disorders of the peripheral and central nervous system are widespread, and for many of these conditions effective therapeutic interventions are lacking.

WO 97/34618 relates to the treatment of central nervous system injuries by intracisternal or intravenous administration of polypeptide growth factors, such as fibroblast growth factor.>

The present invention provides oncomodulin for use in treating a neurological disorder in a subject suffering therefrom by promoting neuronal survival and regeneration or axonal outgrowth in the subject, the use comprising administering to the subject a therapeutically effective amount of the oncomodulin.

The present invention further provides a pharmaceutical composition comprising oncomodulin and a pharmaceutically acceptable carrier, packed with instructions for use of the pharmaceutical composition for promoting neuronal survival and regeneration or axonal outgrowth in a subject suffering from a neurological disorder.

The present invention further provides use of oncomodulin in the manufacture of a composition or compositions for treating a neurological disorder in a subject suffering therefrom.

*Summary of the Invention*

In one aspect, the present invention enables a method which includes administering to a subject a therapeutically effective amount of a macrophage-derived factor, oncomodulin, thereby producing a neurosalutary effect in the

subject. The and uses described herein may The methods and uses described herein may further include administering to a subject a cAMP modulator or an axogenic factor.

In one aspect, the macrophage-derived factor, oncomodulin, is administered to a subject in accordance with the present invention such that the factor is brought into contact with neurons of the central nervous system of the subject. For example, the factor may be administered into the cerebrospinal fluid of the subject into the intrathecal space by introducing the factor into a cerebral ventricle, the lumbar area, or the cisterna magna. In such circumstances, the macrophage-derived factor can be administered locally to cortical neurons or retinal ganglion cells to produce a neurosalutary effect.

In certain embodiments, the pharmaceutically acceptable formulation provides sustained delivery, providing effective amounts of the macrophage-derived factor to a subject for at least one week, or in other embodiments, at least one month, after the pharmaceutically acceptable formulation is initially administered to the subject. Approaches for achieving sustained delivery of a formulation of the invention include the use of a slow release polymeric capsule, a bioerodible matrix, or an infusion pump that disperses the factor or other therapeutic compound of the invention. The infusion pump may be implanted subcutaneously, intracranially, or in other locations as would be medically desirable. In certain embodiments, the therapeutic factors or compositions of the invention would be dispensed by the infusion pump via a catheter either into the cerebrospinal fluid, or to a site where local delivery was desired, such as a site of neuronal injury or a site of neurodegenerative changes.

The present invention enables a method which includes administering to a subject a therapeutically effective amount of oncomodulin in combination with a therapeutically effective amount of an axogenic factor, thereby producing a neurosalutary effect in the subject.

The present invention enables a method which includes administering to a subject a therapeutically effective amount of oncomodulin in combination with a therapeutically effective amount of an axogenic factor and a therapeutically effective amount of a cAMP modulator, thereby producing a neurosalutary effect in the subject.

The present invention enables a method which includes administering to a subject a therapeutically effective amount of oncomodulin in combination with an effective amount of AF-1, AF-2 or inosine, thereby producing a neurosalutary effect in the subject.

In one embodiment, the pharmaceutical composition may further include a cAMP modulator and/or an axogenic factor, such as AF-1, AF-2 or a purine such as inosine.

Other features and advantages of the invention will be apparent from the following detailed description, and from the claims.

*Detailed Description*

Intraocular injections that impinge upon the lens initiate a set of cellular changes that include macrophage infiltration, astrocyte stimulation, and increased expression of the growth-associated protein GAP-43 in retinal ganglion cells. Subsequently, retinal ganglion cells show improved survival and unprecedented levels of axonal growth into the normally prohibitive environment of the optic nerve. Similar results were obtained using the macrophage activator zymosan instead of lens injury. A macrophage-derived factor such as oncomodulin or FGF-β, with or without one or more adjunctive endogenous or exogenous axogenic factors, can also stimulate axonal outgrowth.

As used herein, the term "macrophage-derived factor" includes any factor derived from a macrophage that has the ability to produce a neurosalutary effect in a subject. Macrophage-derived factors include, but are not limited to, peptides such as oncomodulin and TGF-β.

As used herein, a "neurosalutary effect" means a response or result favorable to the health or function of a neuron, of a part of the nervous system, or of the nervous system generally. Examples of such effects include improvements in the ability of a neuron or portion of the nervous system to resist insult, to regenerate, to maintain desirable function, to grow or to survive. The phrase "producing a neurosalutary effect" includes producing or effecting such a response or improvement in function or resilience within a component of the nervous system. For example, examples of producing a neurosalutary effect would include stimulating axonal outgrowth after injury to a neuron; rendering a neuron resistant to apoptosis; rendering a neuron resistant to a toxic compound such as β-amyloid, ammonia, or other neurotoxins; reversing age-related neuronal atrophy or loss of function; or reversing age-related loss of cholinergic innervation.

The term "axogenic factor" includes any factor that has the ability to stimulate axonal regeneration from a neuron. Examples of axogenic factors include AF-1 and AF-2 as described in, for example, Schwalb et al. (1996) Neuroscience 72(4):901-10; Schwalb *et al.*, *id.*; and U.S. Patent No.: 5,898,066 . Other examples of axogenic factors include purines, such as inosine, as described in, for example, PCT application No. PCT/US98/03001 and Benowitz et al. (1999) Proc. Natl. Acad Sci. 96(23):13486-90 .

The term "cAMP modulator" includes any compound which has the ability to modulate the amount, production, concentration, activity or stability of cAMP in a cell, or to modulate the pharmacological activity of cellular cAMP. cAMP modulators may act at the level of adenylate cyclase, upstream of adenylate cyclase, or downstream of adenylate cyclase, such as at the level of cAMP itself, in the signaling pathway that leads to the production of cAMP. Cyclic AMP modulators may act inside the cell, for example at the level of a G-protein such as Gi, Go, Gq, Gs and Gt, or outside the cell, such as at the level of an extra-cellular receptor such as a G-protein coupled receptor. Cyclic AMP modulators include activators of adenylate cyclase such as forskolin; non-hydrolyzable analogues of cAMP including 8-bromo-cAMP, 8-chloro-cAMP, or dibutyryl cAMP (db-cAMP); isoprotenol; vasoactive intestinal peptide; calcium ionophores; membrane depolarization; macrophage-derived factors that stimulate cAMP; agents that stimulate macrophage activation such as zymosan or IFN-γ; phosphodiesterase inhibitors such as pentoxifylline and theophylline; specific phosphodiesterase IV (PDE IV) inhibitors; and beta 2-adrenoreceptor agonists such as salbutamol. The term cAMP modulator also includes compounds which inhibit cAMP production, function, activity or stability, such as phosphodiesterases, such as cyclic nucleotide phosphodiesterase 3B. cAMP modulators which inhibit cAMP production, function, activity or stability are known in the art and are described in, for example, Nano et al. (2000) Pflugers Arch 439(5):547-54 .

"Phosphodiesterase IV inhibitor" refers to an agent that inhibits the activity of the enzyme phosphodiesterase IV. Examples of phosphodiesterase IV inhibitors are known in the art and include 4-arylpyrrolidinones, such as rolipram, nitraquazone, denbufylline, tibenelast,CP-80633 and quinazolinediones such as CP-77059.

"Beta-2 adrenoreceptor agonist" refers to an agent that stimulates the beta-2 adrenergic receptor. Examples of beta-2 adrenoreceptor agonists are known in the art and include salmeterol, fenoterol and isoproterenol.

The term "administering" to a subject includes dispensing, delivering or applying an active compound in a pharmaceutical formulation to a subject by any suitable route for delivery of the active compound to the desired location in the subject, including delivery by either the parenteral or oral route, intramuscular injection, subcutaneous/intradermal injection, intravenous injection, buccal administration, transdermal delivery and administration by the rectal, colonic, vaginal, intranasal or respiratory tract route.

As used herein, the language "contacting" is intended to include both *in vivo* or *in vitro* methods of bringing a compound of the invention into proximity with a neuron such that the compound can exert a neurosalutary effect on the neuron.

As used herein, the term "effective amount" includes an amount effective, at dosages and for periods of time necessary, to achieve the desired result, such as sufficient to produce a neurosalutary effect in a subject. An effective amount of an active compound as defined herein may vary according to factors such as the disease state, age, and weight of the subject, and the ability of the active compound to elicit a desired response in the subject. Dosage regimens may be adjusted to provide the optimum therapeutic response. An effective amount is also one in which any toxic or detrimental effects of the active compound are outweighed by the therapeutically beneficial effects.

A therapeutically effective amount or doasage of an active may range from about 0.001 to 30 mg/kg body weight, with other ranges of the invention including about 0.01 to 25 mg/kg body weight, about 0.1 to 20 mg/kg body weight, about 1 to 10 mg/kg, 2 to 9 mg/kg, 3 to 8 mg/kg, 4 to 7 mg/kg, and 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an active compound can include a single treatment or a series of treatments. In one example, a subject is treated with an active compound in the range of between about 0.1 to 20 mg/kg body weight, one time per week for between about 1 to 10 weeks, alternatively between 2 to 8 weeks, between about 3 to 7 weeks, or for about 4, 5, or 6 weeks. It will also be appreciated that the effective dosage of an active compound used for treatment may increase or decrease over the course of a particular treatment.

The term "subject" is intended to include animals. In particular embodiments, the subject is a mammal, a human or nonhuman primate, a dog, a cat, a horse, a cow or a rodent.

"Neurological disorder" is intended to include a disease, disorder, or condition which directly or indirectly affects the normal functioning or anatomy of a subject's nervous system. Elements of the nervous system subject to disorders which may be effectively treated with the compounds and methods of the invention include the central, peripheral, somatic, autonomic, sympathetic and parasympathetic components of the nervous system, neurosensory tissues within the eye, ear, nose, mouth or other organs, as well as glial tissues associated with neuronal cells and structures. Neurological disorders may be caused by an injury to a neuron, such as a mechanical injury or an injury due to a toxic compound, by the abnormal growth or development of a neuron, or by the misregulation (such as downregulation or upregulation) of an activity of a neuron. Neurological disorders can detrimentally affect nervous system functions such as the sensory function (the ability to sense changes within the body and the outside environment); the integrative function (the ability to interpret the changes); and the motor function (the ability to respond to the interpretation by initiating an action such as a muscular contraction or glandular secretion). Examples of neurological disorders include traumatic or toxic injuries to peripheral or cranial nerves, spinal cord or to the brain, cranial nerves, traumatic brain injury, stroke, cerebral aneurism, and spinal cord injury. Other neurological disorders include cognitive and neurodegenerative disorders such as Alzheimer's disease, dementias related to Alzheimer's disease (such as Pick's disease), Parkinson's and other Lewy diffuse body diseases, senile dementia, Huntington's disease, Gilles de la Tourette's syndrome, multiple sclerosis, amyotrophic lateral sclerosis, hereditary motor and sensory neuropathy (Charcot-Marie-Tooth disease), diabetic neuropathy, progressive supranuclear palsy, epilepsy, and Jakob-Creutzfieldt disease. Autonomic function disorders include hypertension and sleep disorders. Also to be treated with compounds and methods of the invention are neuropsychiatric disorders such as depression, schizophrenia, schizoaffective disorder, Korsakoff's psychosis, mania, anxiety disorders, or phobic disorders, learning or memory disorders (such as amnesia and age-related memory loss), attention deficit disorder, dysthymic disorder, major depressive disorder, mania, obsessive-compulsive disorder, psychoactive substance use disorders, anxiety, phobias, panic disorder, bipolar affective disorder, psychogenic pain syndromes, and eating disorders. Other examples of neurological disorders include injuries to the nervous system due to an infectious disease (such as meningitis, high fevers of various etiologies, HIV, syphilis, or post-polio syndrome) and injuries to the nervous system due to electricity (including contact with electricity or lightning, and complications from electro-convulsive psychiatric therapy). The developing brain is a target for neurotoxicity in the developing central nervous system through many stages of pregnancy as well as during infancy and early childhood, and the methods of the invention may be utilized in preventing or treating neurological deficits in embryos or fetuses in utero, in premature infants, or in children with need of such treatment, including those with neurological birth defects. Further neurological disorders include, for example, those listed in Harrison's Principles of Internal Medicine (Braunwald et al., McGraw-Hill, 2001) and in the American Psychiatric Association's Diagnostic and Statistical Manual of Mental Disorders DSM-IV (American Psychiatric Press, 2000) .

The term "stroke" is art recognized and is intended to include sudden diminution or loss of consciousness, sensation, and voluntary motion caused by rupture or obstruction (for example, by a blood clot) of an artery of the brain.

"Traumatic brain injury" is art recognized and is intended to include the condition in which, a traumatic blow to the head causes damage to the brain or connecting spinal cord, often without penetrating the skull. Usually, the initial trauma can result in expanding hematoma, subarachnoid hemorrhage, cerebral edema, raised intracranial pressure, and cerebral hypoxia, which can, in turn, lead to severe secondary events due to low cerebral blood flow.

The term "outgrowth" includes the process by which axons or dendrites extend from a neuron. The outgrowth can result in a new neuritic projection or in the extension of a previously existing cellular process. Axonal outgrowth may include linear extension of an axonal process by 5 cell diameters or more. Neuronal growth processes, including neuritogenesis, can be evidenced by GAP-43 expression detected by methods such as immunostaining. "Modulating axonal outgrowth" means stimulating or inhibiting axonal outgrowth to produce salutary effects on a targeted neurological disorder.

The term "CNS neurons" is intended to include the neurons of the brain, the cranial nerves and the spinal cord.

Various aspects of the invention are described in further detail in the following subsections:

Pharmaceutically Acceptable Formulations

Pharmaceutical compositions and packaged formulations comprising a macrophage-derived factor and a pharmaceutically acceptable carrier are also provided by the invention. These pharmaceutical compositions may also include an axogenic factor and/or a cAMP modulator.

In the method and use described herein, the macrophage-derived factor, oncomodulin, optionally in conjunction with an axogenic factor and/or a cAMP modulator, can be administered in a pharmaceutically acceptable formulation. Such pharmaceutically acceptable formulation may include the macrophage-derived factor as well as a pharmaceutically acceptable carrier(s) and/or excipient(s). As used herein, "pharmaceutically acceptable carrier" includes any and all solvents, dispersion media, coatings, antibacterial and anti fungal agents, isotonic and absorption delaying agents, and the like that are physiologically compatible. For example, the carrier can be suitable for injection into the cerebrospinal fluid. Excipients include pharmaceutically acceptable stabilizers and disintegrants. The present invention pertains to any pharmaceutically acceptable formulations, including synthetic or natural polymers in the form of macromolecular complexes, nanocapsules, microspheres, or beads, and lipid-based formulations including oil-in-water emulsions, micelles, mixed micelles, synthetic membrane vesicles, and resealed erythrocytes.

In one embodiment, the pharmaceutically acceptable formulations comprise a polymeric matrix. The terms "polymer" or "polymeric" are art-recognized and include a structural framework comprised of repeating monomer units which is capable of delivering a macrophage-derived factor such that treatment of a targeted condition, such as a neurological disorder, occurs. The terms also include co-polymers and homopolymers such as synthetic or naturally occurring. Linear polymers, branched polymers, and cross-linked polymers are also meant to be included.

For example, polymeric materials suitable for forming the pharmaceutically acceptable formulation employed in the present invention, include naturally derived polymers such as albumin, alginate, cellulose derivatives, collagen, fibrin, gelatin, and polysaccharides, as well as synthetic polymers such as polyesters (PLA, PLGA), polyethylene glycol, poloxomers, polyanhydrides, and pluronics. These polymers are biocompatible with the nervous system, including the central nervous system, they are biodegradable within the central nervous system without producing any toxic byproducts of degradation, and they possess the ability to modify the manner and duration of the active compound release by manipulating the polymer's kinetic characteristics. As used herein, the term "biodegradable" means that the polymer will degrade over time by the action of enzymes, by hydrolytic action and/or by other similar mechanisms in the body of the subject As used herein, the term "biocompatible" means that the polymer is compatible with a living tissue or a living organism by not being toxic or injurious and by not causing an immunological rejection. Polymers can be prepared using methods known in the art.

The polymeric formulations can be formed by dispersion of the active compound within liquefied polymer, as described in U.S. Pat. No. 4,883,666 or by such methods as bulk polymerization, interfacial polymerization, solution polymerization and ring polymerization as described in Odian G., Principles of Polymerization and ring opening polymerization, 2nd ed., John Wiley & Sons, New York, 1981 . The properties and characteristics of the formulations are controlled by varying such parameters as the reaction temperature, concentrations of polymer and the active compound, the types of solvent used, and reaction times.

The active therapeutic compound can be encapsulated in one or more pharmaceutically acceptable polymers, to form a microcapsule, microsphere, or microparticle, terms used herein interchangeably. Microcapsules, microspheres, and microparticles are conventionally free-flowing powders consisting of spherical particles of 2 millimeters or less in diameter, usually 500 microns or less in diameter. Particles less than 1 micron are conventionally referred to as nanocapsules, nanoparticles or nanospheres. For the most part, the difference between a microcapsule and a nanocapsule, a microsphere and a nanosphere, or microparticle and nanoparticle is size; generally there is little, if any, difference between the internal structure of the two. In one aspect of the present invention, the mean average diameter is less, than about 45 $\mu$m, preferably less than 20 $\mu$m, and more preferably between about 0.1 and 10 $\mu$m.

In another embodiment, the pharmaceutically acceptable formulations comprise lipid-based formulations. Any of the known lipid-based drug delivery systems can be used in the practice of the invention. For instance, multivesicular liposomes, multilamellar liposomes and unilamellar liposomes can all be used so long as a sustained release rate of the encapsulated active compound can be established. Methods of making controlled release multivesicular liposome drug delivery systems are described in PCT Application Serial Nos. US96/11642, US94/12957 and US94/04490 .

The composition of the synthetic membrane vesicle is usually a combination of phospholipids, usually in combination with steroids, especially cholesterol. Other phospholipids or other lipids may also be used.

Examples of lipids useful in synthetic membrane vesicle production include phosphatidylglycerols, phosphatidylcholines, phosphatidylserines, phosphatidylethanolamines, sphingolipids, cerebrosides, and gangliosides, with preferable embodiments including egg phosphatidylcholine, dipalmitoylphosphatidylcholine, distearoylphosphatidylcholine, dioleoylphosphatidylcholine, dipalmitoylphosphatidylglycerol, and dioleoylphosphatidylglycerol.

In preparing lipid-based vesicles containing an active compound such variables as the efficiency of active compound encapsulation, lability of the active compound, homogeneity and size of the resulting population of vesicles, active compound-to-lipid ratio, permeability, instability of the preparation, and pharmaceutical acceptability of the formulation should be considered.

Prior to introduction, the formulations can be sterilized, by any of the umerous available techniques of the art, such as with gamma radiation or electron beam sterilization.

Administration of the Pharmaceutically Acceptable Formulation

The pharmaceutically acceptable formulations of the invention are administered such that the active compound comes into contact with a subject's nervous system to thereby produce a neurosalutary effect. Both local and systemic administration of the formulations are contemplated by the invention. Desirable features of local administration include achieving effective local concentrations of the active compound as well as avoiding adverse side effects from systemic administration of the active compound. In one embodiment, the active compound is administered by introduction into the cerebrospinal fluid of the subject. In certain aspects of the invention, the active compound is introduced into a cerebral ventricle, the lumbar area, or the cisterna magna. In another aspect, the active compound is introduced locally, such as into the site of nerve or cord injury, into a site of pain or neural degeneration, or intraocularly to contact neuroretinal cells.

The pharmaceutically acceptable formulations can be suspended in aqueous vehicles and introduced through conventional hypodermic needles or using infusion pumps.

In one embodiment, the active compound formulation described herein is administered to the subject in the period from the time of, for example, an injury to the CNS up to about 100 hours after the injury has occurred, for example within 24, 12, or 6 hours from the time of injury.

In another embodiment of the invention, the active compound formulation is administered into a subject intrathecally. As used herein, the term "intrathecal administration" is intended to include delivering an active compound formulation directly into the cerebrospinal fluid of a subject, by techniques including lateral cerebroventricular injection through a burrhole or cisternal or lumbar puncture or the like (described in Lazorthes et al. Advances in Drug Delivery Systems and Applications in Neurosurgery, 143-192 and Omaya et al., Cancer Drug Delivery, 1: 169-179 ). The term "lumbar region" is intended to include the area between the third and fourth lumbar (lower back) vertebrae. The term "cisterna magna" is intended to include the area where the skull ends and the spinal cord begins at the back of the head. The term "cerebral ventricle" is intended to include the cavities in the brain that are continuous with the central canal of the spinal cord. Administration of an active compound to any of the above mentioned sites can be achieved by direct injection of the active compound formulation or by the use of infusion pumps. Implantable or external pumps and catheter may be used.

For injection, the active compound formulation of the invention can be formulated in liquid solutions, preferably in physiologically compatible buffers such as Hank's solution or Ringer's solution. In addition, the active compound formulation may be formulated in solid form and re-dissolved or suspended immediately prior to use. Lyophilized forms are also included. The injection can be, for example, in the form of a bolus injection or continuous infusion (such as using infusion pumps) of the active compound formulation.

In one embodiment of the invention, the active compound formulation is administered by lateral cerebroventricular injection into the brain of a subject, preferably within 100 hours of when an injury (resulting in a condition characterized by aberrant axonal outgrowth of central nervous system neurons) occurs (such as within 6, 12, or 24 hours of the time of the injury). The injection can be made, for example, through a burr hole made in the subject's skull. In another embodiment, the formulation is administered through a surgically inserted shunt into the cerebral ventricle of a subject, preferably within 100 hours of when an injury occurs (such as within 6, 12 or 24 hours of the time of the injury). For example, the injection can be made into the lateral ventricles, which are larger, even though injection into the third and fourth smaller ventricles can also be made. In yet another embodiment, the active compound formulation is administered by injection into the cisterna magna, or lumbar area of a subject, preferably within 100 hours of when an injury occurs (such as within 6, 12, or 24 hours of the time of the injury).

An additional means of administration to intracranial tissue involves application of compounds of the invention to the olfactory epithelium, with subsequent transmission to the olfactory bulb and transport to more proximal portions of the brain. Such administration can be by nebulized or aerosolized prerparations.

In another embodiment of the invention, the active compound formulation is administered to a subject at the site of injury, preferably within 100 hours of when an injury occurs (such as within 6, 12, or 24 hours of the time of the injury).

Duration and Levels of administration

In a preferred embodiment of the method of the invention, the active compound is administered to a subject for an extended period of time to produce a neurosalutary effect, such as effect modulation of axonal outgrowth. Sustained contact with the active compound can be achieved by, for example, repeated administration of the active compound over a period of time, such as one week, several weeks, one month or longer. More preferably, the pharmaceutically acceptable formulation used to administer the active compound provides sustained delivery, such as "slow release" of the active compound to a subject. For example, the formulation may deliver the active compound for at least one, two, three, or four weeks after the pharmaceutically acceptable formulation is administered to the subject. Preferably, a subject to be treated in accordance with the present invention is treated with the active compound for at least 30 days (either by repeated administration or by use of a sustained delivery system, or both).

As used herein, the term "sustained delivery" is intended to include continual delivery of the active compound *in vivo* over a period of time following administration, preferably at least several days, a week, several weeks, one month or longer. Sustained delivery of the active compound can be demonstrated by, for example, the continued therapeutic effect of the active compound over time (such as sustained delivery of the macrophage-derived factor can be demonstrated by continued production of a neurosalutary effect in a subject). Alternatively, sustained delivery of the active compound may be demonstrated by detecting the presence of the active compound *in vivo* over time.

Preferred approaches for sustained delivery include use of a polymeric capsule, a minipump to deliver the formulation, a bioerodible implant, or implanted transgenic autologous cells (as described in U.S. Patent No. 6,214,622). Implantable infusion pump systems (such as Infusaid; see such as Zierski, J. et al. (1988) Acta Neurochem. Suppl. 43: 94-99; Kanoff, R.B. (1994) J Am. Osteopath. Assoc. 94:487-493) and osmotic pumps (sold by Alza Corporation) are available in the art. Another mode of administration is via an implantanble, externally programmable infusion pump. Suitable infusion pump systems and reservoir systems are also described in U.S. Patent No. 5, 368,562 by Blomquist and U.S. Patent No. 4,731,058 by Doan, developed by Pharmacia Deltec Inc.

It is to be noted that dosage values may vary with the severity of the condition to be alleviated. It is to be further understood that for any particular subject, specific dosage regimens should be adjusted over time according to the individual need and the professional judgment of the person administering or supervising the administration of the active compound and that dosage ranges set forth herein are exemplary only and are not intended to limit the scope or practice of the claimed invention.

The invention, in another embodiment, provides a pharmaceutical composition consisting essentially of the macrophage derived factor, oncomodulin, and a pharmaceutically acceptable carrier, as well as methods of use thereof to modulate axonal outgrowth by contacting CNS neurons with the composition. By the term "consisting essentially of" is meant that the pharmaceutical composition does not contain any other modulators of neuronal growth such as, for example, nerve growth factor (NGF). In one embodiment, the pharmaceutical composition of the invention can be provided as a packaged formulation. The packaged formulation may include a pharmaceutical composition of the invention in a container and printed instructions for administration of the composition for producing a neurosalutary effect in a subject having a neurological disorder. Use of the macrophage derived factor, oncomodulin, in the manufacture of a medicament for modulating the axonal outgrowth of neurons is also encompassed by the invention.

## *In vitro* Treatment of CNS Neurons

Neurons derived from the central or peripheral nervous system can be contacted with the macrophage-derived factor, oncomodulin, (alone or in combination with an axogenic factor and/or a cAMP modulator) *in vitro* to modulate axonal outgrowth *in vitro*. Accordingly, neurons can be isolated from a subject and grown *in vitro*, using techniques well known in the art, and then treated in accordance with the present invention to modulate axonal outgrowth. Briefly, a neuronal culture can be obtained by allowing neurons to migrate out of fragments of neural tissue adhering to a suitable substrate (such as a culture dish) or by disaggregating the tissue, such as mechanically or enzymatically, to produce a suspension of neurons. For example, the enzymes trypsin, collagenase, elastase, hyaluronidase, DNase, pronase, dispase, or various combinations thereof can be used. Methods for isolating neuronal tissue and the disaggregation of tissue to obtain isolated cells are described in Freshney, Culture of Animal Cells, A Manual of Basic Technique, Third Ed., 1994.

Such cells can be subsequently contacted with the macrophage-derived factor, oncomodulin, (alone or in combination with an axogenic factor and/or a cAMP modulator) in amounts and for a duration of time as described above. Once modulation of axonal outgrowth has been achieved in the neurons, these cells can be re-administered to the subject, such as by implantation.

## Screening Assays

The ability of a macrophage-derived factor (alone or in combination with an axogenic factor and/or a cAMP modulator) to produce a neurosalutary effect in a subject may be assessed using any of a variety of known procedures and assays. For example, the ability of a macrophage-derived factor (alone or in combination with an axogenic factor and/or a cAMP modulator) to re-establish neural connectivity and/or function after an injury, such as a CNS injury, may be determined histologically (either by slicing neuronal tissue and looking at neuronal branching, or by showing cytoplasmic transport of dyes). The ability of compounds of the invention to re-establish neural connectivity and/or function after an injury, such as a CNS injury, may also be assessed by monitoring the ability of the macrophage-derived factor (alone or in combination with an axogenic factor and/or a cAMP modulator) to fully or partially restore the electroretinogram after damage to the neural retina or optic nerve; or to fully or partially restore a pupillary response to light in the damaged eye.

Other tests that may be used to determine the ability of a macrophage-derived factor (alone or in combination with an axogenic factor and/or a cAMP modulator) to produce a neurosalutary effect in a subject include standard tests of neurological function in human subjects or in animal models of spinal injury (such as standard reflex testing, urologic tests, urodynamic testing, tests for deep and superficial pain appreciation, proprioceptive placing of the hind limbs, ambulation, and evoked potential testing). In addition, nerve impulse conduction can be measured in a subject, such as by measuring conduct action potentials, as an indication of the production of a neurosalutary effect.

Animal models suitable for use in the assays of the present invention include the rat model of partial transection (described in Weidner et al. (2001) Proc. Natl. Acad. Sci. USA 98:3513-3518). This animal model tests how well a compound can enhance the survival and sprouting of the intact remaining fragment of an almost fully-transected cord. Accordingly, after administration of the macrophage-derived factor (alone or in combination with an axogenic factor and/or a cAMP modulator) these animals may be evaluated for recovery of a certain function, such as how well the rats may manipulate food pellets with their forearms (to which the relevant cord had been cut 97%).

Another animal model suitable for use in the assays of the present invention includes the rat model of stroke (described in Kawamata et al. (1997) Proc. Natl. Acad. Sci. USA 94(15):8179-8184). This paper describes in detail various tests that may be used to assess sensorimotor function in the limbs as well as vestibulomotor function after an injury. Administration to these animals of the compounds of the invention can be used to assess whether a given compound, route of administration, or dosage provides a neurosalutary effect, such as increasing the level of function, or increasing the rate of regaining function or the degree of retention of function in the test animals.

Standard neurological evaluations used to assess progress in human patients after a stroke may also be used to evaluate the ability of a macrophage-derived factor (alone or in combination with an axogenic factor and/or a cAMP modulator) to produce a neurosalutary effect in a subject. Such standard neurological evaluations are routine in the medical arts, and are described in, for example, "Guide to Clinical Neurobiology" Edited by Mohr and Gautier (Churchill Livingstone Inc. 1995).

For assessing function of the peripheral nervous system, standard tests include electromyography, nerve conduction velocity measurements, evoked potentials assessment and upper/lower extremity somato-sensory evoked potentials.. Electromyography tests record the electrical activity in muscles, and is used to assess the function of the nerves and muscles. The electrode is inserted into a muscle to record its electrical activity. It records activity during the insertion, while the muscle is at rest, and while the muscle contracts. The nerve conduction velocity test evaluates the health of the peripheral nerve by recording how fast an electrical impulse travels through it. A peripheral nerve transmits information between the spinal cord and the muscles. A number of nervous system diseases may reduce the speed of this impulse. Electrodes placed on the skin detect and record the electrical signal after the impulse travels along the nerve. A second stimulating electrode is sends a small electrical charge along the nerve;the time between the stimulation and response will be recorded to determine how quickly and thoroughly the impulse is sent.

Standard tests for function of the cranial nerves, as known to those skilled in the neurological medical art, include facial nerve conduction studies; orbicularis oculi reflex studies (blink reflex studies); trigeminal-facial nerve reflex evaluation as used in focal facial nerve lesions, Bell's palsy, trigeminal neuralgia and atypical facial pain; evoked potentials assessment; visual, brainstem and auditory evoked potential measurements; thermo-diagnostic small fiber testing; and computer-assisted qualitative sensory testing.

The invention is further illustrated by the following examples, which should not be construed as further limiting.

### Examples

The following materials and methods were used in the Examples described herein.

Optic nerve surgery and intraocular injections

Surgical procedures were based upon those described previously (Berry et al., 1996), and were approved by the Children's Hospital Animal Care and Use Committee. Adult male Fisher rats (Charles River Laboratories, Wilmington, MA), 250-350 g, were kept in a pathogen-controlled environment in standard cages and were allowed to feed *ad libitum.* Animals were sedated by Methoxyflurane inhalation (Schering-Plough, Union, NJ) and anesthetized with an intraperitoneal injection of Ketamine (60-80 mg/kg: Phoenix Pharmaceutical, St. Joseph, MO) and Xylazine (10-15 mg/kg: Bayer, Shawnee Mission, KA). After the head was shaved, rats were positioned in a stereotaxic apparatus (Kopf Instruments, Tujunga, CA) and a 1-1.5 cm incision was made in the skin above the right orbit. Under microscopic illumination, the lacrimal glands and extraocular muscles were resected to expose 3-4 mm of the optic nerve. The epineurium was slit open along the long axis and the nerve was crushed 2 mm behind the eye with angled jeweler's forceps (Dumont # 5) for 10 seconds, avoiding injury to the ophthalmic artery. Nerve injury was verified by the appearance of a clearing at the crush site, while the vascular integrity of the retina was evaluated by fundoscopic examination. Cases in which the vascular integrity of the retina was in question were excluded from the study. For intraocular injections, the globe was retracted with a mosquito snap to expose its posterior aspect. In some cases, injections were made through the sclera and retina with a 30G needle 1-2 mm superior to the optic nerve head, inserting the tip of the needle perpendicular to the axis of the nerve to a depth of 2 mm without infringing upon the lens (minimally invasive injection); in other cases, the tip of the needle was bent at a 90° angle and inserted into the eye 2 mm above the nerve head, perpendicular to the sclera, to intentionally puncture the lens surface. Lens injury was confirmed by direct visualization through the cornea; further verification of lens injury was an opacification that occurred within 1 week. Injection volumes were 5 μl using saline as a vehicle; in some cases, we examined the effects of needle puncture alone without injections. Survival times ranged from 1 to 40 days.

Groups included controls with no surgery (N = 3), animals with lens puncture but no nerve crush (N=11), animals with nerve crush and either no intraocular surgery (N = 24) or a single puncture of the lens (N = 24); animals with nerve crush and an anterior lens puncture at the limbus (N = 4) or with multiple posterior punctures of the lens (N = 3); animals with nerve crush and a single injection (via a posterior approach) of either recombinant rat CNTF (5 μg/ml, Alamone Labs, Jerusalem, Israel, N = 5; or 10 μg/ml, Promega Labs, N = 5), an anti-rat CNTF polyclonal antibody (20 μg/ml, R&D Systems, Minneapolis, MN, N = 4), basic fibroblast growth factor (5 μg/ml, kindly provided by Dr. Patricia D'Amore, Children's Hospital Boston, MA: N = 3) anti-bovine basic FGF (1-5 mg/ml, Upstate Biotechnology, Lake Placid, NY, N = 4), anti-BDNF (R&D, mouse monoclonal, 5 mg/ml, N = 4) or 0.9% NaCl (N = 4). Animals showing signs of intravitreal hemorrhage after puncture were excluded.

Sciatic nerve implants

Pre-degenerated peripheral nerve fragments were obtained by performing a crush injury on the peroneal branch of the sciatic nerve in 4 rats. Four days later, rats were killed with an overdose of Ketamine plus Xylazine, and the portion of the nerve distal to the crush site was dissected out. As described previously (Berry et al., 1996), sections of sciatic nerve c. 1 mm in length were implanted into host animals which had undergone optic nerve surgery as described above (N = 5). Fragments were inserted by cutting a small radial slit through the sclera and implanting a single piece of tissue into the vitreous, taking care to avoid injuring the lens.

Preparation for histology

At survival times ranging from 1 to 40 days, animals were given a lethal overdose of anesthesia and perfused through the heart with ice-cold PBS with heparin (10.000U in 100 ml) followed by 4% paraformaldehyde in PBS (100 ml). Eyes with nerve segments up to the optic chiasm still attached were dissected free from connective tissue, postfixed overnight in 4% paraformaldehyde (4° C) and transferred to a 30% sucrose solution overnight with constant rocking (4° C). Frozen sections (15 μm thickness) were cut longitudinally on a cryostat, thaw-mounted onto coated glass slides (Superfrost Plus, Fisher), and stored at -80° C until further use.

Immunohistochemistry

Sections were stained with antibodies to visualize either the neuronal growth-associated protein GAP-43; glial fibrillary acidic protein (GFAP); ED-1, a marker for activated cells of monocyte lineage; or myelin basic protein (MBP). GAP-43 was visualized using the IgG fraction of an antibody prepared in sheep (Benowitz et al., 1988), followed by either a biotin- or fluorescein-conjugated secondary antibody. In the former case, sections were preincubated with 0.3% $H_2O_2$ in 100% methanol (30 min), blocked with 5% rabbit serum in TBS, pH 7.4 (1 hr), and incubated in the primary antibody at a 1:50,000 dilution (in TBS containing 300 mM NaCl, 2% BSA, and 0.1% Tween-20: $TBS_2T$) overnight (4° C, constant rocking). Sections were rinsed (3X over a 4 hr period in $TBS_2T$), incubated in biotinylated rabbit anti-sheep IgG (1:250 in $TBS_2T$: Vector Labs, Burlingame, CA), rinsed 3X, and reacted with avidin-biotin-HRP complex for 1 hour (following the manufacturer's protocol: Vector Labs) followed by diaminobenzidine (DAB) enhanced with $NiCl_2$ (Vector Labs). In cases in which GAP-43 was visualized by immunofluorescence, similar conditions were used except that the primary antibody was diluted 1:2500 and the secondary antibody was a fluorescein-conjugated anti-sheep IgG made in rabbit (1:500, Vector Labs). In cases in which GAP-43 was visualized together with other antigens, we used a mouse monoclonal anti-GAP-43 antibody (clone 9-1E12, 1:250 dilution, Boehringer-Mannheim) followed by a fluorescein-conjugated anti-mouse IgG made in horse (Vector, 1:500).

Immunofluorescent sections were covered using Vectashield (Vector) as a mounting medium. To visualize changes in Müller cells, we used a rabbit anti-GFAP antibody (Sigma, 1:7500) and a biotinylated goat anti-rabbit IgG (1:500). Reactive macrophages were detected with the ED-1 antibody (Serotec, 1:200 dilution) and biotinylated horse anti-mouse IgG (Vector, 1:500). Myelin was visualized in the optic nerve using a rabbit anti-MBP antibody (1:25, Zymed Labs) followed by a Texas red-conjugated goat anti-rabbit IgG (1:500, Vector).

Quantitation of axon growth

Axon growth was quantified by counting the number of GAP-43-positive axons extending 0.5 mm and 1 mm from the end of the crush site in 4 sections per case. The cross-sectional width of the nerve was measured at the point at which the counts were taken, and was used to calculate the number of axons per mm nerve width. The number of axons/mm was then averaged over the 4 sections. $\Sigma a_d$, the total number of axons extending distance $d$ in a nerve having a radius of $r$, was estimated by summing over all sections having a thickness t (= 15 $\mu$m):

$$\Sigma a_d = \pi r^2 \times [average\ axons/mm]/t$$

Anterograde labeling

We used cholera toxin B fragment (CTB) as an anterograde tracer to verify that axons visualized in the distal optic nerve originated in RGCs. Animals that underwent nerve crush, either with or without lens puncture, were injected with CTB (2.5 $\mu$g/$\mu$l in 5 $\mu$l PBS) 20 days after the original surgery. Animals were euthanized and prepared for histology the following day as described above. Slide-mounted sections were reacted with an antibody to CTB (made in goat; List Biological Lab, 1:40,000 dilution), followed by a rabbit anti-goat IgG secondary antibody (Vector, 1:500 dilution). In some cases, GAP-43 and CTB were examined together, using a monoclonal anti-GAP-43 antibody made in mouse and the goat anti-CTB antibody (1:250), followed with the appropriate secondary antibodies conjugated to fluorescein and Texas red, respectively (Vector, 1:500).

Quantitation of cell survival

For cell survival studies, RGCs were retrogradely labeled with Fluorogold (Molecular Probes) 7 days prior to nerve crush. Rats were anesthetized as above, a midline incision was made in the scalp, and a bone flap was opened above the occipital cortex. Posterior cortex was vacuum-aspirated and multiple injections of Fluorogold (5 $\mu$g/ml in PBS containing 1% DMSO, 1 $\mu$l per injection) were made into the superior colliculi (depth c. 1 mm). Gelfoam (1 mm$^3$, Upjohn) soaked in the same Fluorogold solution was inserted over the colliculus. One week later, animals received an optic nerve crush combined with either a lens puncture or a minimally invasive intraocular injection. Normal controls (N = 5) were labeled to obtain baseline values of RGC density.
Twenty one days after nerve crush and intraocular injections (i.e., 28 days after Fluorogold labeling), animals were euthanized with an overdose of Ketamine plus Xylazine and the retinas were dissected without fixation. After making a radial slit, each retina was placed on a nylon filter attached to a microscope slide, overlaid with filter paper soaked in 4% paraformaldehyde (in PBS), and held down with weights on the edges for 1 hr. Retinas were then removed from the filters, flat-mounted, and covered using Vectashield. Under fluorescent illumination (X200 magnification), 6 regions, radially distributed at 1 and 2 mm from the optic nerve head, were counted for labeled RGCs using a 10x 10 grid (0.16 mm$^2$). Counts were averaged across the 6 regions.

Western blotting for GAP-43 and GFAP

Fourteen days after optic nerve crush, unfixed retinas were freshly dissected and solubilized in 100 $\mu$l of 2X SDS-PAGE sample buffer (O'Farrell, 1975). Samples were balanced for protein content and separated by SDS-PAGE in mini-gels (Bio-Rad). Proteins were transferred to PVDF membranes (0.45 $\mu$m pore, Millipore) and probed using antibodies to either GAP-43 or GFAP. In the former case, the staining protocol closely followed that used for tissue, except that the concentration of monoclonal anti-GAP-43 antibody (Boehringer) was 1:1000; in the case of GFAP, the primary antibody was used at a concentration of 1:5000. Secondary antibodies were HRP-conjugated; immunoreactivity was detected with ECL reagent (Amersham Life Science) and fluorography.

Macrophage activation

Several methods were attempted to stimulate macrophages in the eye without puncturing the lens. These included injecting interferon-$\gamma$ (IFN-$\gamma$, GibcoBRL, 5000 U in 5 $\mu$l; N = 5) at a dosage sufficient to activate monocytes throughout the nervous system (Sethna and Lampson, 1991); or Zymosan (625 $\mu$g in 5 $\mu$l, Sigma; N = 5), a yeast cell wall preparation (Fitch et al., 1999; Lombard et al., 1994; Ross and Vetvicka, 1993; Stewart and Weir, 1989). We also introduced activated macrophages, obtained from donor animals by injecting $Ca^{2+}$ - and $Mg^{2+}$ - free buffer containing 0.025% trypsin and 2 mM EDTA into the peritoneal cavity as described (Smith and Hale, 1997); after 3 min, the cavity was opened, fluid was removed and added to DMEM (Sigma) containing 1% fetal bovine serum (Gemini). Cells were collected by centrifugation, resuspended in the same medium, plated in culture dishes, and incubated 4 hr. After washing off nonadherent cells, the remaining cells were removed with trypsin, added to culture media, collected by centrifugation, and washed with saline. The presence of activated macrophages was verified by staining cells with ED-1 and OX-42 antibodies (Serotec). Approximately $10^5$ macrophages were injected into a host vitreous, with care taken to avoid injuring the lens (N = 4). To suppress macrophage activation after lens puncture, we used the tripeptide MIF (estd. final conc. in eye 50 $\mu$M; Sigma, N = 6), Ciglitazone (estd. final conc. in eye, 75 $\mu$M; Biomol, Plymouth Meeting, PA, N = 4), or prostaglandin J2 (estd. final conc. in eye, 80 $\mu$M; Calbiochem, N = 3).

## EXAMPLE 1: Axonal Outgrowth

In mature mammals, retinal ganglion cells (RGCs) are unable to regenerate their axons after optic nerve injury and soon undergo apoptotic cell death. However, as demonstrated in the following example, a small puncture wound to the lens enhanced RGC survival and enabled these cells to regenerate their axons into the normally inhibitory environment of the optic nerve. Even when the optic nerve was intact, lens injury stimulated macrophage infiltration into the eye, Müller cell activation, and increased GAP-43 expression in ganglion cells across the entire retina. In contrast, axotomy, either alone or combined with intraocular injections that did not infringe upon the lens, caused only a minimal change in GAP-43 expression in RGCs and a minimal activation of the other cell types. Combining nerve injury with lens puncture led to a 8-fold increase in RGC survival and a 100-fold increase in the number of axons regenerating beyond the crush site. The effects of lens puncture could not be explained by changes in the levels of several candidate growth factors tested. However, macrophage activation was shown to play a key role, because intraocular injections of Zymosan, a yeast cell wall preparation, stimulated monocytes in the absence of lens injury and induced RGCs to regenerate their axons into the distal optic nerve.

Because RGCs only express GAP-43 during axon outgrowth, probes for this protein enable one to visualize RGCs in a growth state (Berry et al., 1996; Doster et al., 1991; Meiri et al., 1986; Moya et al., 1988; Schaden et al., 1994). In animals having a nerve crush combined with lens puncture, numerous GAP-43-positive axons grew past the injury into the distal optic nerve. Control optic nerves showed no staining at all. Animals with nerve crush but with either no intraocular injections or intraocular injections that did not infringe upon the lens showed some GAP-43 immunostaining in the proximal region of the nerve (see below) and in the neuroma that forms at the injury site, but almost no growth beyond this point. Quantitatively, animals with optic nerve crush alone (N = 5) averaged $4 \pm 3$ axons (mean $\pm$ SEM) extending 0.5 mm past the crush site, and none at 1 mm; animals in which the nerve was crushed but which received minimally invasive injections (N = 7) had only slightly more growth ($22 \pm 9$ axons at 0.5 mm, and $6 \pm 3$ at 1 mm). Relative to the latter group, animals in which the nerve was crushed and the lens punctured showed a nearly 100-fold increase in growth ($1791 \pm 232$ axons at 0.5 mm, and $933 \pm 162$ axons at 1 mm distal to the crush site: N= 6. The difference between the latter group and controls with nerve crush plus minimally invasive injections was highly significant (p < 0.001 at both 0.5 and 1.0 mm).

The number of axons reaching 0.5 or 1 mm past the injury site rose continuously over the first 3 weeks. By 40 days, however, the number declined, suggesting that GAP-43 expression in RGCs had diminished or that some of the axons that had been present at 3 weeks degenerated.

Anterograde labeling with cholera toxin

Anterograde labeling afforded a more rigorous way to demonstrate that axons distal to the crush site arose from RGCs. For these studies, CTB was injected into the posterior chamber 1 day prior to sacrificing animals, then immunohistochemistry was carried out to detect CTB in the optic nerve. The pattern of CTB staining closely resembled that for GAP-43. After nerve crush without lens puncture, CTB-positive axons were detected proximal to the injury site but not beyond it; with nerve crush plus lens puncture, many CTB-positive axons appeared in the distal nerve. Double-labeling revealed that axonal elements growing beyond the crush site contained both antigens, and in some cases, intense double-labeling was observed in structures resembling growth cones. In 4 CTB-labeled animals having optic nerve crush and lens puncture, $903 \pm 54$ axons were ounted at 0.5 mm with CTB staining vs. $1422 \pm 259$ GAP-43-

positive axons at the same distance; a similar labeling ratio was seen at 1 mm. The discrepancy between the numbers of CTB- and GAP-43-positive axons may be due to a failure of RGCs distant from the injection site to take up CTB.

Although CNS myelin is inhibitory to axon growth, RGC axons appear to regenerate through myelin-rich areas of the nerve after lens puncture. This is apparent in double-immunostained sections in which growing axons were labeled with antibodies to GAP-43 and myelin with antibodies to MBP. Because the 15 μm sections are thicker than the axons, it remains possible that the axons may be growing through myelin-free zones within the nerve, though no gaps in the MBP staining pattern are apparent. However, the pattern of myelin staining in the nerve 21 days after crush does have a reticulated appearance that differs from the continuous, striated staining found in the normal optic nerve.

In the normal rat retina, GAP-43 immunostaining is limited to the processes of dopaminergic amacrine cells in the inner plexiform layer (Kapfhammer et al., 1997). RGCs are unstained, as are their axons within the optic nerve. Twenty one days after optic nerve crush without lens injury, RGCs remained unlabeled, and few GAP-43-positive axons appeared in the optic nerve proximal to the crush site. In contrast, when nerve crush was accompanied by lens injury, there was a dramatic increase in the immunostaining of RGCs and in their axons within the overlying fiber layer and in the optic nerve proximal to the crush site. The number of GAP-43-positive fibers extending up to the injury site greatly exceeds the number that continues past this point. Surprisingly, even without nerve damage, lens injury stimulated RGCs to express GAP-43 across the full extent of the retina, despite the fact that these cells' axons were not damaged. Correspondingly, some normal axons in the undamaged optic nerve showed GAP-43 immunostaining after lens injury.

GAP-43 was not detected in RGCs 24 hours after nerve crush with lens puncture (not shown), but became visible by day 3 and intensified by day 7. By 21 days, GAP-43 levels were high throughout the retina. Animals with nerve crush alone showed only a small, transient increase in GAP-43 expression at 7 days that could no longer be detected at 21 days. The effect of lens puncture alone on RGCs was evident on day 7 and, as mentioned above, remained high at 21 days.

The effect of combining nerve injury and lens puncture was confirmed on western blots of the retina and proximal optic nerve segment. These studies were carried out 14 days after surgery, a time at which the transient upregulation of GAP-43 expression from nerve injury alone has mostly subsided (Doster et al., 1991; Wodarczyk et al., 1997). Western blots did not reveal an appreciable change in overall retinal GAP-43 levels 14 da after a nerve crush with a minimally invasive intraocular injection or after lens injury without nerve crush. This is presumably due to the fact that the GAP-43 changes in RGCs are modest, whereas the levels in amacrine are considerable and unchanging. However, the effect of combining nerve crush and lens injury was clearly evident in the retina and was much more dramatic in the optic nerve, where the only source of GAP-43 are RGC axons, in which levels of the protein are changing radically.

Retinal ganglion cell survival

To evaluate cell survival, Fluorogold was used to retrogradely label RGCs 1 week prior to surgery. In the normal, intact visual pathway, Fluorogold labeled numerous RGCs, which are characteristically round or oval cells, 12-15 μm across. Quantitation revealed a cell density of $1806 \pm 54$ RGCs/mm$^2$ (N = 14 cases), similar to previously reported results (Clarke et al., 1998; Koeberle and Ball, 1998; Mansour-Robaey et al., 1994; Mey and Thanos, 1993). In animals with nerve injury alone, or nerve injury combined with a minimally invasive injection into the eye, very few RGCs remained 3 weeks after surgery: in the latter case, $59 \pm 21$ RGCs/mm$^2$ were counted, i.e., only 3% of the original number (N = 7). At the same time, numerous small, brightly fluorescent, spiny cells with multiple processes appeared. These latter cells have been described previously in the injured retina, and represent activated microglia (Clarke et al., 1998; Koeberle and Ball, 1998; Sawai et al., 1996; Thanos et al., 1993). When nerve injury was accompanied by lens puncture, RGC survival increased about 8-fold, i.e., 24% of the original number of RGCs remained 3 weeks after surgery ($430 \pm 38$ RGCs/mm$^2$: N = 7). Microglia were still evident, but in much smaller numbers, and were readily distinguished from the RGCs by their morphology and location in a different optical plane.

Astrocyte reaction

In normal rats, GFAP staining is restricted to the dense network of astrocytic processes in the innermost segment of the retina, and this pattern did not change appreciably 7 or 21 days after nerve crush alone. Minimally invasive injections that did not infringe upon the lens caused a very limited GFAP upregulation restricted to the region of the needle track. However, puncturing the lens, even without injuring the nerve, stimulated GFAP expression in Müller cells across the full extent of the retina. This change was detectable by 3 days, became pronounced by 7 days, and persisted for at least 3 weeks. Crushing the optic nerve in addition to puncturing the lens did not increase GFAP expression in Müller cells beyond the level induced by lens injury alone. These results are confirmed on western blots: lens puncture alone increased overall GFAP levels in the retina, whereas nerve crush alone had a smaller effect. The effect of combining nerve injury with lens puncture was similar to that of lens puncture alone.

Macrophage reaction

The normal retina shows few, if any, ED-1-positive macrophages, and this was not altered by nerve crush alone nor by intraocular injections that did not infringe upon the lens, except in the vicinity of the needle track. In contrast, lens injury led to widespread macrophage infiltration across the whole extent of the retina whether or not the nerve was crushed. This first became apparent at 3 days and intensified at 7 days; combining lens puncture with nerve injury induced about the same level of macrophage infiltration as lens puncture alone. Thus, the onset of the macrophage reaction correlates with the changes seen in RGCs and in Müller cells, with all three being induced strongly by lens injury but only minimally by nerve crush. Table I summarizes the pattern of changes seen in the eye 7-14 days after various experimental conditions. At 21 days, whereas GAP-43 expression in the retina and GFAP expression in Müller cells remained high, the number of ED-1 positive cells subsided considerably. As demonstrated by Fluorogold labeling, there are still numerous microglia in the retina at 21 days, particularly after nerve crush alone, but these do not stain with the ED-1 antibody.

**Table I.** Summary of changes resulting from nerve injury and/or lens puncture

| cellular change | control | nerve crush | lens puncture | crush + puncture |
|---|---|---|---|---|
| GAP-43+ RGCs | - | +/- | + | +++ |
| GFAP+ Müller cells | - | +/- | ++ | ++ |
| ED-1+ macrophages | - | - | +++ | +++ |

Immunizing animals against myelin has recently been reported to enable injured corticospinal tract axons to regenerate through the dorsal funiculus of the rat's spinal cord after injury (Huang et al., 1999). Since lens puncture causes a strong inflammatory reaction in the eye, we investigated whether it also stimulates an immune response that may contribute to the changes in RGC survival and axon regeneration. Sera were obtained from normal controls, or 7 days after surgery from animals having either a nerve crush with a minimally invasive injection in the eye, or nerve injury combined with lens puncture (N = 3 in each group). These sera were used to stain western blots of proteins from the retina and optic nerve derived from normal animals and from animals 7 days after nerve crush. The results showed no differences in the staining patterns obtained with the different antibodies. It is also predicted that if circulating antibodies were contributing to the regeneration observed in our studies, lens puncture might have an effect that would carry over to the contralateral optic nerve if it were also injured. Results from such studies show very little elevation of GAP-43 in the retina or proximal optic nerve contralateral to the eye receiving a lens puncture when both optic nerves were injured; only the side with the lens puncture exhibited elevated levels of GAP-43. Similarly, immunohistochemistry revealed no axon growth past the injury site on the side in which the optic nerve was injured but the eye only received a minimally invasive injection. This result suggests that GAP-43 induction is related to local effects that ensue from lens puncture, rather than from systemically circulating agents, such as antibodies.

Within the nerve, numerous macrophages were seen in the vicinity of the crush site within 7 days of injury, and by 14 days, these cells became dispersed along the length of the nerve; by 21 days, their distribution narrowed to the vicinity of the crush site. Puncturing the lens without crushing the nerve induced no macrophage reaction in the nerve, and combining lens puncture with nerve crush did not augment the response beyond the level seen after nerve crush alone. By 7 days, a massive cavity has formed at the crush site in all cases, presenting a formidable barrier to axon growth (Battisti et al., 1995; Fitch et al., 1999; McKeon et al., 1991).

Defined growth factors

Puncture wounds to the posterior chamber of the eye cause a selective induction of CNTF and basic FGF mRNA (Cao et al., 1997; Faktorovich et al., 1992; Wen et al., 1995). CNTF induces RGCs to extend axons in dissociated cell culture (Jo et al., 1999) and through a peripheral nerve graft *in vivo* (Cui et al., 1999). Based upon these studies, the ability of CNTF to mediate the effects of lens puncture on RGCs was investigated. Using minimally invasive injections, CNTF was introduced at concentrations up to 1 μg/ml, approximately 1000 times the $ED_{50}$ required to stimulate axon outgrowth from rat RGCs in culture (Jo et al., 1999; Meyer-Franke et al., 1995). Axon growth was examined at 14 days, a time point at which growth past the crush site is clear-cut after lens puncture, but before the effects of the single injection might have subsided. Intraocular injections of CNTF that did not infringe upon the lens had no effect. Whether the RGC changes that result from lens puncture could be diminished with anti-CNTF antibodies was also investigated (R&D Systems, 20 μg/ml, a quantity sufficient to neutralize 80% of the activity of 1 ng/ml of CNTF). No changes were observed. Neutralizing antibodies to BDNF or basic FGF likewise failed to diminish the number of axons measured at 0.5 or 1 mm distal to the injury site after lens puncture. However, anti-BDNF antibody treatment doubled the length of the longest regenerating axon measured distal to the injury site (relative to cases with lens puncture plus nerve crush: t = 3.67, p < 0.01, df = 8). No other treatment significantly increased axon length.

Multiple punctures

Whether the regenerative changes obtained after a single lens injury could be augmented by multiple punctures was also investigated. This was examined by either making 10 punctures on the same day as the nerve injury or 5 punctures at 3 day intervals beginning the same day as the nerve crush. Both of these treatments only diminished axon growth, perhaps because of generalized trauma to the retina. Thus, despite the fact that a single puncture initially affects a very small region of the lens, it may elicit a maximal response.

Macrophage activation mimics the effect of lens puncture

To determine whether macrophages mediate the effect of lens puncture on RGC survival and axon regeneration, several methods were used to activate macrophages without encroaching on the lens. Of these, the greatest effect was achieved by injecting Zymosan, a yeast cell wall preparation, to augment the modest macrophage response that occurs after minimally invasive intraocular injections. Zymosan stimulated an extensive macrophage response in the eye, and this was paralleled by an upregulation of GFAP in Müller cells and of GAP-43 in RGCs; under these conditions, extensive axon regeneration was observed past the site of nerve injury.

After establishing that a macrophage-derived factor was what caused the stimulation of neuronal survival and axon growth, chromatographic separation was performed to isolate the active molecule(s). One potential factor was isolated, sequences and found to correspond to the protein Oncomodulin (described in, for example, Ritzler J. M. et al. (1992) Genomics 12:567-572). When tested in culture, oncomodulin stimulated retinal ganglion cells to regenerated their axons. Another macrophage-derived factor, FGF-$\beta$, was also found to stimulate regeneration of retinal ganglion cells in culture. The effect of TGF-$\beta$ synergized with AF-1 and elevated cAMP.

The vitreous is highly inhibitory to inflammation (Osusky et al., 1996); in conformity with this, it was not possible to elicit a sustained macrophage reaction by injecting IFN-$\gamma$ or by introducing activated peritoneal macrophages. Correlated with the absence of monocyte activation were an absence of GAP-43 upregulation in RGCs and a failure of axons to regenerate past the crush site in the nerve. IFN-$\gamma$ did, however, stimulate GFAP expression in Müller cells. On the other hand, none of the inhibitors used (MIF, Ciglitazone, prostaglandin J2) diminished macrophage activation after lens puncture, nor did they alter GAP-43 expression.

Summary

In mature mammals, RGCs are unable to regrow injured axons and soon undergo apoptotic death. These well-known events are a paradigm of regenerative failure in the CNS, and may mimic pathophysiological sequelae that underlie degenerative diseases such as glaucoma. However, as shown here, if the lens is injured, RGCs show increased survival and regenerate their axons into the distal optic nerve.

The pro-regenerative effects of lens puncture appear to be mediated through activated macrophages. Within 3 days of nerve crush with lens puncture, ED-1-positive monocytes appear across the entire retina. This is paralleled by an upregulation of GAP-43 in RGCs and of GFAP in Müller cells; these changes all intensify by 7 days and remain high for another week. Macrophage activity then begins to decline, but the changes in Müller cells and RGCs persist. In contrast, nerve injury, either alone or combined with a minimally invasive intraocular injection, induces only minor macrophage activation, and is soon followed by massive RGC death.

To investigate whether macrophages play a causative role in stimulating RGC survival and regeneration, we used several methods to stimulate monocytes without infringing upon the lens. Zymosan, a yeast membrane suspension that activates the mannose and $\beta$ -glucan lectin-binding site of the CR3 $\beta_2$-integrin receptors, resulted in massive macrophage infiltration into the eye. This was accompanied by a dramatic increase in GAP-43 expression in RGCs and axon regeneration beyond the injury site. The vitreous is normally suppressive to inflammation; this has been attributed to high levels of hyaluronic acid and TGF-$\beta$II. Thus, lens puncture may release agents that overcome the anti-inflammatory influences that normally prevail intravitreally. Our results indicate that the effects of lens puncture and macrophage activation are local, and are not mediated through circulating antibodies.

Activated monocytes release a host of cytokines and growth factors that can stimulate neurons directly or indirectly via glial stimulation (Giulian (1993) Glia, 7:102-110; Kreutzberg (1996) Trends Neurosci. 19:312-318). In the rat striatum, puncture wounds stimulate microglia that express BDNF and promote the infiltration of macrophages that express GDNF; these two growth factors are likely to contribute to the survival and outgrowth of dopaminergic neurons that occurs after puncture wounds (Batchelor et al. (1999) J. Neurosci. 19:1708-1716). BDNF and GDNF also affect RGC survival after axotomy (Di Polo et al. (1998) Proc. Natl. Acad. Sci. USA 95:3978-3983; Koeberle and Ball (1998) Vision Res. 38:1505-1515; Mansour-Robaey et al. (1994) Proc. Natl. Acad. Sci. USA 91:1632-1636; Mey and Thanos (1993) Brain Res. 602:304-317; Sawai et al. (1996) J. Neurosci. 16:3887-3894), and could play a role here. However, neutralizing anti-BDNF antibodies did not diminish the positive effects of lens injury, but augmented the length of axon growth into the distal optic nerve. This unanticipated effect on axon length may be a consequence of suppressing the effects of BDNF upon axon branching (Cohen-Cory (1999) J. Neurosci. 19:9996-10003; Jo et al., (1999) Neuroscience 89:579-591; Lom and Cohen-Cory (1999) J. Neurosci. 19:9928-9938; Mansour-Robaey et al. (1994) Proc. Natl. Acad. Sci. USA 91:1632-1636; Sawai et al. (1996) J. Neurosci. 16:3887-3894). In terms of glial contributions, activated Müller cells increase CNTF expression (Ju et al. (1999) Neuroreport 10:419-422), and CNTF can stimulate RGC survival (Mey and Thanos (1993) Brain Res. 602:304-317; Meyer-Franke et al. (1995) Neuron 15:805-819) and axon regeneration (Jo et al. (1999) Neuroscience 89:579-591; Cui et al. (1999) Invest. Ophthalmol. Vis. Sci. 40:760-766). However, intraocular injections of CNTF did not stimulate axon regeneration in the absence of lens puncture, and anti-CNTF antibodies did not diminish the effects of lens injury. Traumatic injury to the eye also increases mRNA for bFGF (Cao et al. (1997) Exp. Eye Res. 65:241-248; Faktorovich et al. (1992) J. Neurosci. 12:3554-3567; Wen et al. (1995) J. Neurosci. 15:7377-7385), but again, anti-bFGF antibodies did not diminish the effect of lens puncture. A number of studies on trophic factors in the eye found that control injections enhanced RGC survival (Koeberle and Ball (1998) Vision Res. 38: 1505-1515; Mansour-Robaey et al. (1994) Proc. Natl. Acad. Sci. USA 91:1632-1636). These effects may have resulted from lens injury, since in our hands, intraocular injections that did not infringe upon the lens had little effect on RGCs.

***Equivalents***

Those skilled in the art will recognize, or be able to ascertain using no more than routine experimentation, many equivalents to the specific embodiments of the invention described herein. Such equivalents are intended to be encompassed by the following claims.

**Claims**

1.  Oncomodulin for use in treating a neurological disorder in a subject suffering therefrom by promoting neuronal survival and regeneration or axonal outgrowth in the subject, the use comprising administering to the subject a therapeutically effective amount of the oncomodulin.

2.  The oncomodulin for use according to claim 1, wherein the use further comprises administering to the subject a cAMP modulator

3.  The oncomodulin for use according to claim 2, wherein said cAMP modulator is selected from non-hydrolyzable cAMP analogues, adenylate cyclase activators, macrophage-derived factors that stimulate cAMP, macrophage activators, calcium ionophores, phosphodiesterase inhibitors, specific phosphodiesterase IV inhibitors, beta2-adrenoreceptor inhibitors and vasoactive intestinal peptide.

4.  The oncomodulin for use according to claim 1, wherein the use further comprises administering to the subject an axogenic factor.

5.  The oncomodulin for use according to claim 4, wherein the axogenic factor is AF-1.

6.  The oncomodulin for use according to claim 4, wherein the axogenic factor is inosine.

7.  The oncomodulin for use according to claim 1, wherein the neurons are retinal ganglion cells.

8.  The oncomodulin for use according to claim 1, wherein the use comprises administering the oncomodulin by introduction into a region of neuronal injury; introduction into the cerebrospinal fluid of the subject; introduction to the subject intrathecally; or introduction into a region selected from a cerebral ventricle, the lumbar area, and the cisterna magna of the subject.

9.  The oncomodulin for use according to claim 1, wherein the oncomodulin is administered to the subject in a pharmaceutically acceptable formulation.

10.  The oncomodulin for use according to claim 9, wherein the pharmaceutically acceptable formulation is selected from a dispersion system; a lipid-based formulation; a formulation comprising a polymeric matrix; a formulation contained within a minipump; a formulation providing sustained delivery of the macrophage-derived factor for at least one week after the pharmaceutically acceptable formulation is administered to the subject; and a formulation providing sustained delivery of the macrophage-derived factor for at least one month after the pharmaceutically acceptable formulation is administered to the subject.

11. The oncomodulin for use according to claim 10, wherein the pharmaceutically acceptable formulation comprises a liposome formulation.

12. The oncomodulin for use according to claim 10, wherein the pharmaceutically acceptable formulation comprises a multivesicular liposome formulation.

13. The oncomodulin for use according to claim 1, wherein the subj ect is a mammal.

14. The oncomodulin for use according to claim 13, wherein the mammal is a human.

15. The oncomodulin for use according to claim 1, wherein the neurological disorder is caused by injury to a neuron.

16. The oncomodulin for use according to claim 15, wherein the injury is a mechanical injury or injury due to a toxic compound.

17. The oncomodulin for use according to claim 1, wherein the neurological disorder is a cognitive or neurodegenerative disorder.

18. The oncomodulin for use according to claim 17, wherein the cognitive or neurodegenerative disorder is selected from Alzheimer's disease, Parkinson's and other Lewy diffuse body diseases, senile dementia, Huntington's disease, Gilles de la Tourette's syndrome, multiple sclerosis, amyotrophic lateral sclerosis, hereditary motor and sensory neuropathy, diabetic neuropathy, progressive supranuclear palsy, epilepsy, and Jakob-Creutzfieldt disease.

19. The oncomodulin for use according to claim 18, wherein the epilepsy is posttraumatic epilepsy.

20. The oncomodulin for use according to claim 1, wherein the neurological disorder is a spinal cord injury.

21. The oncomodulin for use according to claim 20, wherein the spinal cord injury is **characterized by** one or more of: monoplegia, diplegia, paraplegia, hemiplegia and quadriplegia.

22. A pharmaceutical composition comprising oncomodulin and a pharmaceutically acceptable carrier, packed with instructions for use of the pharmaceutical composition for promoting neuronal survival and regeneration or axonal outgrowth in a subject suffering from a neurological disorder.

23. The pharmaceutical composition of claim 22, further comprising one or more of: a cAMP modulator; and an axogenic factor.

24. The pharmaceutical composition of claim 23, wherein the axogenic factor is AF-1 or inosine.

25. Use of oncomodulin in the manufacture of a composition or compositions for treating a neurological disorder in a subject suffering therefrom.

26. The use of claim 25, wherein the composition is administrable to the subject after making a first assessment of a nervous system function in the subject and before making a second assessment of the nervous system function in the subject.

27. The use of claim 26, wherein the nervous system function is a sensory function, cholinergic innervation, or a vestibulomotor function.

28. The use of claim 25, wherein the neurological disorder is caused by injury to a neuron.

29. The use of claim 28, wherein the injury is a mechanical injury or injury due to a toxic compound.

30. The use of claim 25, wherein the neurological disorder is a cognitive or neurodegenerative disorder.

31. The use of claim 30, wherein the cognitive or neurodegenerative disorder is selected from Alzheimer's disease, Parkinson's and other Lewy diffuse body diseases, senile dementia, Huntington's disease, Gilles de la Tourette's syndrome, multiple sclerosis, amyotrophic lateral sclerosis, hereditary motor and sensory neuropathy, diabetic neuropathy, progressive supranuclear palsy, epilepsy, and Jakob-Creutzfieldt disease.

32. The use of claim 31, wherein the epilepsy is posttraumatic epilepsy.

33. The use of claim 25, wherein the neurological disorder is a spinal cord injury.

34. The use of claim 33, wherein the spinal cord injury is **characterized by** one or more of: monoplegia, diplegia, paraplegia, hemiplegia and quadriplegia.

**Patentansprüche**

1. Oncomodulin zur Verwendung zum Behandeln einer neurologischen Erkrankung in einem Wesen, welches an ihr leidet, durch Fördern des neuronalen Überlebens und der Regenerierung oder des axonalen Auswachsens in dem Wesen, wobei die Verwendung umfasst, dass dem Wesen eine therapeutisch effektive Menge des Oncomodulins verabreicht wird.

2. Oncomodulin zur Verwendung nach Anspruch 1, wobei die Verwendung weiterhin umfasst, dass dem Wesen ein cyclo-AMP-Modulator verabreicht wird.

3. Oncomodulin zur Verwendung nach Anspruch 2, wobei der cyclo-AMP-Modulator aus nicht-hydrolysierbaren cyclo-AMP-Analoga, Adenylatcyclaseaktivatoren, von Makrophagen abgeleiteten Faktoren, welche cyclo-AMP stimulieren, Makrophagenaktivatoren, Calciumionophoren, Phosphodiesteraseinhibitoren, spezifischen Phosphodiesterase-IV-Inhibitoren, beta-2-Adrenorezeptorinhibitoren und vasoaktiven Intestinalpeptiden ausgewählt ist.

4. Oncomodulin zur Verwendung nach Anspruch 1, wobei die Verwendung weiterhin umfasst, dass dem Wesen ein axogener Faktor verabreicht wird.

5. Oncomodulin zur Verwendung nach Anspruch 4, wobei der axogene Faktor AF-1 ist.

6. Oncomodulin zur Verwendung nach Anspruch 4, wobei der axogene Faktor Inosin ist.

7. Oncomodulin zur Verwendung nach Anspruch 1, wobei die Neuronen retinale Ganglionzellen sind.

8. Oncomodulin zur Verwendung nach Anspruch 1, wobei die Verwendung umfasst, dass das Oncomodulin durch Einbringung in einen Bereich neuronaler Verletzung, durch Einbringung in den Liquor des Wesens, dem Wesen durch intrathekale Einbringung, oder durch Einbringung in einen Bereich, welcher aus einem Hirnventrikel, dem Lendenwirbelbereich, und der Cisterna magna des Wesens ausgewählt ist, verabreicht wird.

9. Oncomodulin zur Verwendung nach Anspruch 1, wobei das Oncomodulin dem Wesen in einer pharmazeutisch verträglichen Formulierung verabreicht wird.

10. Oncomodulin zur Verwendung nach Anspruch 9, wobei die pharmazeutisch verträgliche Formulierung aus einem Dispersionssystem; einer lipidbasierten Formulierung; einer Formulierung, welche eine Polymermatrix aufweist; einer Formulierung, welche innerhalb einer Minipumpe enthalten ist; einer Formulierung, welche fortwährende Lieferung des von Makrophagen abgeleiteten Faktors mindestens eine Woche lang, nachdem die pharmazeutisch verträgliche Formulierung dem Wesen verabreicht wurde, zur Verfügung stellt; und einer Formulierung, welche fortwährende Lieferung des von Makrophagen abgeleiteten Faktors mindestens einen Monat lang, nachdem die pharmazeutisch verträgliche Formulierung dem Wesen verabreicht wurde, zur Verfügung stellt, ausgewählt ist.

11. Oncomodulin zur Verwendung nach Anspruch 10, wobei die pharmazeutisch verträgliche Formulierung eine Liposomformulierung aufweist.

12. Oncomodulin zur Verwendung nach Anspruch 10, wobei die pharmazeutisch verträgliche Formulierung eine multivesikulare Liposomformulierung aufweist.

13. Oncomodulin zur Verwendung nach Anspruch 1, wobei das Wesen ein Säugetier ist.

14. Oncomodulin zur Verwendung nach Anspruch 13, wobei das Säugetier ein Mensch ist.

15. Oncomodulin zur Verwendung nach Anspruch 1, wobei die neurologische Erkrankung durch eine Verletzung eines Neurons verursacht ist.

16. Oncomodulin zur Verwendung nach Anspruch 15, wobei die Verletzung eine mechanische Verletzung oder eine Verletzung aufgrund einer toxischen Verbindung ist.

17. Oncomodulin zur Verwendung nach Anspruch 1, wobei die neurologische Erkrankung eine kognitive oder neurodegenerative Erkrankung ist.

18. Oncomodulin zur Verwendung nach Anspruch 17, wobei die kognitive oder neurodegenerative Krankheit aus Alzheimerscher Krankheit, Parkinson's und anderen Lewy-Körperdemenzen, seniler Demenz, Chorea Huntington, Tourette-Syndrom, Multipler Sklerose, amyotropher Lateralsklerose, hereditärer motorisch-sensibler Neuropathie, diabetischer Neuropathie, progressiver supranukleärer Blickparese, Epilepsie und Creutzfeldt-Jakob-Krankheit ausgewählt ist.

19. Oncomodulin zur Verwendung nach Anspruch 18, wobei die Epilepsie eine posttraumatische Epilepsie ist.

20. Oncomodulin zur Verwendung nach Anspruch 1, wobei die neurologische Erkrankung eine Rückenmarksverletzung ist.

21. Oncomodulin zur Verwendung nach Anspruch 20, wobei die Rückenmarksverletzung durch eine oder mehr aus Monoplegie, Diplegie, Paraplegie, Hemiplegie und Quadriplegie gekennzeichnet ist.

22. Pharmazeutische Zusammensetzung, welche Oncomodulin und einen pharmazeutisch verträglichen Träger aufweist, welche mit einer Gebrauchsanleitung für die pharmazeutische Zusammensetzung zur Förderung von neuronalem Überleben und Regenerierung oder axonalem Auswachsen in einem Wesen, welches an einer neurologischen Erkrankung leidet, verpackt ist.

23. Pharmazeutische Zusammensetzung nach Anspruch 22, welche darüber hinaus einen oder mehr aus einem cyclo-AMP-Modulator und einem axogenen Faktor aufweist.

24. Pharmazeutische Zusammensetzung nach Anspruch 23, wobei der axogene Faktor AF-1 oder Inosin ist

25. Verwendung von Oncomodulin zur Herstellung einer Zusammensetzung oder von Zusammensetzungen zur Behandlung einer neurologischen Erkrankung in einem Wesen, welches an ihr leidet.

26. Verwendung nach Anspruch 25, wobei die Zusammensetzung dem Wesen verabreichbar ist, nachdem eine erste Beurteilung einer Funktion des Nervensystems in dem Wesen gemacht worden ist, und bevor eine zweite Beurteilung der Funktion des Nervensystems in dem Wesen gemacht wird.

27. Verwendung nach Anspruch 26, wobei die Funktion des Nervensystems eine Sinnesfunktion, eine cholinerge Innervation, oder eine vestibulomotorische Funktion ist.

28. Verwendung nach Anspruch 25, wobei die neurologische Erkrankung durch die Verletzung eines Neurons verursacht ist.

29. Verwendung nach Anspruch 28, wobei die Verletzung eine mechanische Verletzung oder eine Verletzung aufgrund einer toxischen Verbindung ist.

30. Verwendung nach Anspruch 25, wobei die neurologische Erkrankung eine kognitive oder neurodegenerative Erkrankung ist.

**31.** Verwendung nach Anspruch 30, wobei die kognitive oder neurodegenerative Erkrankung aus Alzheimerscher Krankheit, Parkinson's und anderen Lewy-Körperdemenzen, seniler Demenz, Chorea Huntington, Tourette-Syndrom, Multipler Sklerose, amyotropher Lateralsklerose, hereditärer motorisch-sensibler Neuropathie, diabetischer Neuropathie, progressiver supranukleärer Blickparese, Epilepsie, und Creutzfeldt-Jakob-Krankheit, ausgewählt ist.

**32.** Verwendung nach Anspruch 31, wobei die Epilepsie eine posttraumatische Epilepsie ist.

**33.** Verwendung nach Anspruch 25, wobei die neurologische Erkrankung eine Rückenmarksverletzung ist.

**34.** Verwendung nach Anspruch 33, wobei die Rückenmarksverletzung durch eine oder mehr aus Monoplegie, Diplegie, Paraplegie, Hemiplegie und Quadriplegie gekennzeichnet ist.

## Revendications

**1.** Oncomoduline pour une utilisation dans le traitement d'un trouble neurologique chez un sujet souffrant de ce dernier par l'activation de la survie et de la régénérescence neuronale ou de la croissance axonale chez le sujet, l'utilisation comprenant l'administration au sujet d'une quantité thérapeutiquement efficace de l'oncomoduline.

**2.** Oncomoduline pour une utilisation selon la revendication 1, où l'utilisation comprend en outre l'administration au sujet d'un modulateur de l'AMPc.

**3.** Oncomoduline pour une utilisation selon la revendication 2, où ledit modulateur de l'AMPc est choisi parmi des analogues non hydrolysables de l'AMPc, des activateurs de l'adénylate cyclase, des facteurs dérivés des macrophages qui stimulent l'AMPc, désactivateurs des macrophages, des ionophores du calcium, des inhibiteurs de phosphodiestérases, des inhibiteurs spécifiques de la phosphodiestérase IV, des inhibiteurs des récepteurs bêta-2-adrénergiques et le peptide vasoactif intestinal.

**4.** Oncomoduline pour une utilisation selon la revendication 1, où l'utilisation comprend en outre l'administration au sujet d'un facteur axogénique.

**5.** Oncomoduline pour une utilisation selon la revendication 4, où le facteur axogénique est l'AF-1.

**6.** Oncomoduline pour une utilisation selon la revendication 4, où le facteur axogénique est l'inosine.

**7.** Oncomoduline pour une utilisation selon la revendication 1, où les neurones sont des cellules ganglionnaires de la rétine.

**8.** Oncomoduline pour une utilisation selon la revendication 1, où l'utilisation comprend l'administration de l'oncomoduline par introduction dans une région de lésion neuronale ; introduction dans le liquide céphalo-rachidien du sujet ; introduction chez le sujet par voie intrathécale ; ou introduction dans une région choisie parmi un ventricule cérébral, la région lombaire et la grande citerne du sujet.

**9.** Oncomoduline pour une utilisation selon la revendication 1, où l'oncomoduline est administrée au sujet dans une formulation pharmaceutiquement acceptable.

**10.** Oncomoduline pour une utilisation selon la revendication 9, où la formulation pharmaceutiquement acceptable est choisie parmi un système de dispersion ; une formulation à base de lipides ; une formulation comprenant une matrice polymère ; une formulation contenue au sein d'une minipompe ; une formulation fournissant une délivrance prolongée du facteur dérivé des macrophages pendant au moins une semaine après l'administration de la formulation pharmaceutiquement acceptable au sujet ; et une formulation fournissant une libération prolongée du facteur dérivé des macrophages pendant au moins un mois après l'administration de la formulation pharmaceutiquement acceptable au sujet.

**11.** Oncomoduline pour une utilisation selon la revendication 10, où la formulation pharmaceutiquement acceptable comprend une formulation liposomique.

**12.** Oncomoduline pour une utilisation selon la revendication 10, où la formulation pharmaceutiquement acceptable comprend une formulation de liposomes multivésiculaires.

13. Oncomoduline pour une utilisation selon la revendication 1, où le sujet est un mammifère.

14. Oncomoduline pour une utilisation selon la revendication 13, où le mammifère est un être humain.

15. Oncomoduline pour une utilisation selon la revendication 1, où le trouble neurologique est provoqué par la lésion d'un neurone.

16. Oncomoduline pour une utilisation selon la revendication 15, où la lésion est une lésion mécanique ou une lésion due à un composé toxique.

17. Oncomoduline pour une utilisation selon la revendication 1, où le trouble neurologique est un trouble cognitif ou neurodégénératif.

18. Oncomoduline pour une utilisation selon la revendication 17, où le trouble cognitif ou neurodégénératif est choisi parmi la maladie d'Alzheimer, la maladie de Parkinson et d'autres maladies à corps diffus de Lewy, la démence sénile, la maladie de Huntington, le syndrome de Gilles de la Tourette, la sclérose en plaques, la sclérose latérale amyotrophique, une neuropathie motrice et sensorielle héréditaire, une neuropathie diabétique, une paralysie supranucléaire progressive, l'épilepsie et la maladie de Creutzfeld-Jacob.

19. Oncomoduline pour une utilisation selon la revendication 18, où l'épilepsie est une épilepsie post-traumatique.

20. Oncomoduline pour une utilisation selon la revendication 1, où le trouble neurologique est une lésion de la moelle épinière.

21. Oncomoduline pour une utilisation selon la revendication 20, où la lésion de la moelle épinière est **caractérisée par** une ou plusieurs : d'une monoplégie, d'une diplégie, d'une paraplégie, d'une hémiplégie et d'une quadriplégie.

22. Composition pharmaceutique comprenant de l'oncomoduline et un support pharmaceutiquement acceptable, conditionnée avec des instructions d'utilisation de la composition pharmaceutique pour activer la survie et la régénérescence neuronale ou la croissance axonale chez un sujet souffrant d'un trouble neurologique.

23. Composition pharmaceutique selon la revendication 22, comprenant en outre un ou plusieurs : d'un modulateur de l'AMPc ; et d'un facteur axogénique.

24. Composition pharmaceutique selon la revendication 23, dans laquelle le facteur axogénique est l'AF-1 ou l'inosine.

25. Utilisation de l'oncomoduline dans la fabrication d'une composition ou de compositions destinées au traitement d'un trouble neurologique chez un sujet souffrant de ce dernier.

26. Utilisation selon la revendication 25, où la composition peut être administrée au sujet après la réalisation d'une première estimation d'une fonction du système nerveux chez le sujet et avant la réalisation d'une seconde estimation de la fonction du système nerveux chez le sujet.

27. Utilisation selon la revendication 26, où la fonction du système nerveux est une fonction sensorielle, une innervation cholinergique ou une fonction vestibulomotrice.

28. Utilisation selon la revendication 25, où le trouble neurologique est provoqué par la lésion d'un neurone.

29. Utilisation selon la revendication 28, où la lésion est une lésion mécanique ou une lésion due à un composé toxique.

30. Utilisation selon la revendication 25, où le trouble neurologique est un trouble cognitif ou neurodégénératif.

31. Utilisation selon la revendication 30, où le trouble cognitif ou neurodégénératif est choisi parmi la maladie d'Alzheimer, la maladie de Parkinson et d'autres maladies à corps diffus de Lewy, la démence sénile, la maladie de Huntington, le syndrome de Gilles de la Tourette, la sclérose en plaques, la sclérose latérale amyotrophique, une neuropathie motrice et sensorielle héréditaire, une neuropathie diabétique, une paralysie supranucléaire progressive, l'épilepsie et la maladie de Creutzfeld-Jacob.

**32.** Utilisation selon la revendication 31, où l'épilepsie est une épilepsie post-traumatique.

**33.** Utilisation selon la revendication 25, où le trouble neurologique est une lésion de la moelle épinière.

**34.** Utilisation selon la revendication 33, où la lésion de la moelle épinière est **caractérisée par** une ou plusieurs : d'une monoplégie, d'une diplégie, d'une paraplégie, d'une hémiplégie et d'une quadriplégie.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20877800 P
- WO 9734618 A
- US 5898066 A
- US 9803001 W
- US 4883666 A
- US 9611642 W
- US 9412957 B
- US 9404490 B
- US 6214622 B
- US 5368562 A, Blomquist
- US 4731058 A, Doan

**Non-patent literature cited in the description**

- **SCHWALB et al.** *Neuroscience,* 1996, vol. 72 (4), 901-10
- **BENOWITZ et al.** *Proc. Natl. Acad Sci.,* 1999, vol. 96 (23), 13486-90
- **NANO et al.** *Pflugers Arch,* 2000, vol. 439 (5), 547-54
- **BRAUNWALD et al.** Harrison's Principles of Internal Medicine. McGraw-Hill, 2001
- American Psychiatric Association's Diagnostic and Statistical Manual of Mental Disorders DSM-IV. American Psychiatric Press, 2000
- **ODIAN G.** Principles of Polymerization and ring opening polymerization. John Wiley & Sons, 1981
- **LAZORTHES et al.** *Advances in Drug Delivery Systems and Applications in Neurosurgery,* 143-192
- **OMAYA et al.** *Cancer Drug Delivery,* vol. 1, 169-179
- **ZIERSKI, J. et al.** *Acta Neurochem.,* 1988, vol. 43, 94-99
- **KANOFF, R.B.** *J Am. Osteopath. Assoc.,* 1994, vol. 94, 487-493
- **FRESHNEY.** Culture of Animal Cells, A Manual of Basic Technique. 1994
- **WEIDNER et al.** *Proc. Natl. Acad. Sci. USA,* 2001, vol. 98, 3513-3518
- **KAWAMATA et al.** *Proc. Natl. Acad. Sci. USA,* 1997, vol. 94 (15), 8179-8184
- **RITZLER J. M. et al.** *Genomics,* 1992, vol. 12, 567-572
- **GIULIAN.** *Glia,* 1993, vol. 7, 102-110
- **KREUTZBERG.** *Trends Neurosci.,* 1996, vol. 19, 312-318
- **BATCHELOR et al.** *J. Neurosci.,* 1999, vol. 19, 1708-1716
- **DI POLO et al.** *Proc. Natl. Acad. Sci. USA,* 1998, vol. 95, 3978-3983
- **KOEBERLE ; BALL.** *Vision Res.,* 1998, vol. 38, 1505-1515
- **MANSOUR-ROBAEY et al.** *Proc. Natl. Acad. Sci. USA,* 1994, vol. 91, 1632-1636
- **MEY ; THANOS.** *Brain Res.,* 1993, vol. 602, 304-317
- **SAWAI et al.** *J. Neurosci.,* 1996, vol. 16, 3887-3894
- **COHEN-CORY.** *J. Neurosci.,* 1999, vol. 19, 9996-10003
- **JO et al.** *Neuroscience,* 1999, vol. 89, 579-591
- **LOM ; COHEN-CORY.** *J. Neurosci.,* 1999, vol. 19, 9928-9938
- **JU et al.** *Neuroreport,* 1999, vol. 10, 419-422
- **MEYER-FRANKE et al.** *Neuron,* 1995, vol. 15, 805-819
- **CUI et al.** *Invest. Ophthalmol. Vis. Sci.,* 1999, vol. 40, 760-766
- **CAO et al.** *Exp. Eye Res.,* 1997, vol. 65, 241-248
- **FAKTOROVICH et al.** *J. Neurosci.,* 1992, vol. 12, 3554-3567
- **WEN et al.** *J. Neurosci.,* 1995, vol. 15, 7377-7385